Europäisches Patentamt

European Patent Office

Office européen des brevets

(19)

(11) Publication number: **0 064 691**
**B1**

(12) **EUROPEAN PATENT SPECIFICATION**

(45) Date of publication of patent specification: **05.03.86**

(51) Int. Cl.⁴: **G 01 N 31/20, G 01 N 33/52**

(21) Application number: **82103698.5**

(22) Date of filing: **30.04.82**

(54) **Apparatus comprising a magazine retaining multiple flexible test strips and means for removing a single test strip, and method of dispensing such strips using this apparatus.**

(30) Priority: **13.05.81 US 263033**

(43) Date of publication of application:
**17.11.82 Bulletin 82/46**

(45) Publication of the grant of the patent:
**05.03.86 Bulletin 86/10**

(84) Designated Contracting States:
**DE FR GB IT**

(56) References cited:
**US-A-4 218 421**

(73) Proprietor: **MILES LABORATORIES, INC.**
**1127 Myrtle Street**
**Elkhart Indiana 46514 (US)**

(72) Inventor: **Wogoman, Frank**
**209 S. Tuxedo Drive**
**South Bend, IN 46615 (US)**

(74) Representative: **Adrian, Albert, Dr. et al**
**c/o BAYER AG Konzernverwaltung RP**
**Patentabteilung**
**D-5090 Leverkusen 1, Bayerwerk (DE)**

Courier Press, Leamington Spa, England.

**Description**

Field of the Invention

The present invention relates to an apparatus and a method for handling flexible test strips, later on called test devices, used in the determination of a particular constituent in a sample. More particularly, it comprises vacuum means and a pickup head capable of removing one test device at a time from a cartridge or magazine containing multiple test devices.

Background of the Invention

Increasingly, test devices in the form of reagent strips are being used to provide convenient and rapid analysis of various types of samples, including samples of biological, industrial and automotive fluids, wines, and the like. Diagnostic test devices designed for detecting various clinically significant substances or constituents in biological fluids, such as urine and blood, including lysed or unlysed blood, blood plasma and blood serum, have in many cases supplanted prior wet chemistry techniques which were both cumbersome and time consuming. These diagnostic test devices have thus assisted in the fast and accurate diagnosis and treatment of disease.

Conventional test devices generally comprise an absorbent or porous matrix incorporated with indicator reactants, usually of a colorimetric nature. The sample to be tested is contacted with the matrix, such as by momentary immersion where the sample is liquid, and the indicator response is observed after a period of time. For example, in the detection of occult blood in urine a diagnostic test device can be employed which comprises an absorbent paper impregnated with o-tolidine and peroxide. When this test device is wetted with urine containing occult blood, decomposition of the peroxide occurs with the accompanying oxidation of the o-tolidine to provide a color response. This test is sensitive and extremely useful in diagnosing urinary tract disorders.

For ease in handling, the absorbent or porous matrix, sometimes called a "carrier matrix", is advantageously affixed to an insoluble support member such as an organo-plastic strip, e.g., polystyrene, by suitable means such as double faced adhesive tape. Optically transparent substrate material known as TRYCITE, polystyrene film made by Dow Chemical Company, is preferred. The support member normally has a thickness of about .190 mm, a width of about 5 mm and a length which can vary depending on the intended use, the number of reagent areas present, *et cetera*. Currently, test devices are being made by the Ames Division of Miles Laboratories, Inc. which have lengths of about 85.5 mm and about 82.5 mm. Obviously, based on these dimensions and the materials involved, such test devices tend to be small, elongated and flexible in nature. While these test devices are easily manageable during manual handling operations, automated handling is another matter.

With the need for automated equipment employing test devices for determining constituents in fluids, especially for performing multiple diagnostic tests, it is essential that a mechanical system be devised which is capable of rapidly, accurately and reliably handling one test device at a time. The removal of a single test device from a cartridge or magazine containing multiple test devices to permit the transfer of the single test device to another portion of a system for contact with a sample to be tested is, therefore, a key to automating such testing.

In one very special case, i.e., very small test devices which contain a single reagent area, apparatus has been developed for ejecting one test device at a time from a magazine or cartridge containing multiple test devices of a similar nature. Generally, the substrate for such test devices is only slightly larger than the dimensions of the absorbent matrix, which normally measures about 5 mm by about 10 mm. Typically, the magazine or cartridge is spring loaded and the configuration is such that only one test device at a time can be ejected when pressure is applied to one edge of the bottommost test device.

For standard test devices having elongated dimensions as set forth above, however, there is no apparatus available for removing one test device at a time from a cartride or magazine containing other test devices. The system employed in the special case of very small test devices is not effective for use with standard test devices. Accordingly, for both medical and industrial applications which require automation there is a need for fool-proof test device handling apparatus capable of removing a single test device from a cartridge or magazine in order that that single reagent test device can be transferred to another portion of the automated system.

Summary of the Invention

An object of the present invention is to provide means for removing a single test device at a time from a cartridge or magazine containing multiple test devices.

Another object of the present invention is to provide for automated test device handling which is capable of rapidly, accurately and reliably removing a single test device from a cartridge or magazine containing multiple test devices.

In accordance with the present invention, these objects are met by an apparatus according to claim 1 and a method of using it according to claim 8.

Description of the Drawings

Other and further objects, advantages and features of the invention will be apparent to those skilled in the art from the following detailed description thereof, taken in conjunction with the accompanying drawings, in which:

Fig. 1 shows an embodiment of the invention, with portions thereof being shown in cross section;

Fig. 2 is a top view of the apparatus in Fig. 1 taken along lines 2—2;

Fig. 3 is an enlarged cross sectional drawing of the portion of Fig. 1 illustrated by dotted line; and

Figs. 4, 5 and 6 are alternative embodiments of cartridges or magazines for holding test devices which can be utilized in the present invention, said cartridges or magazines being shown in cross section.

## Description of Preferred Embodiments

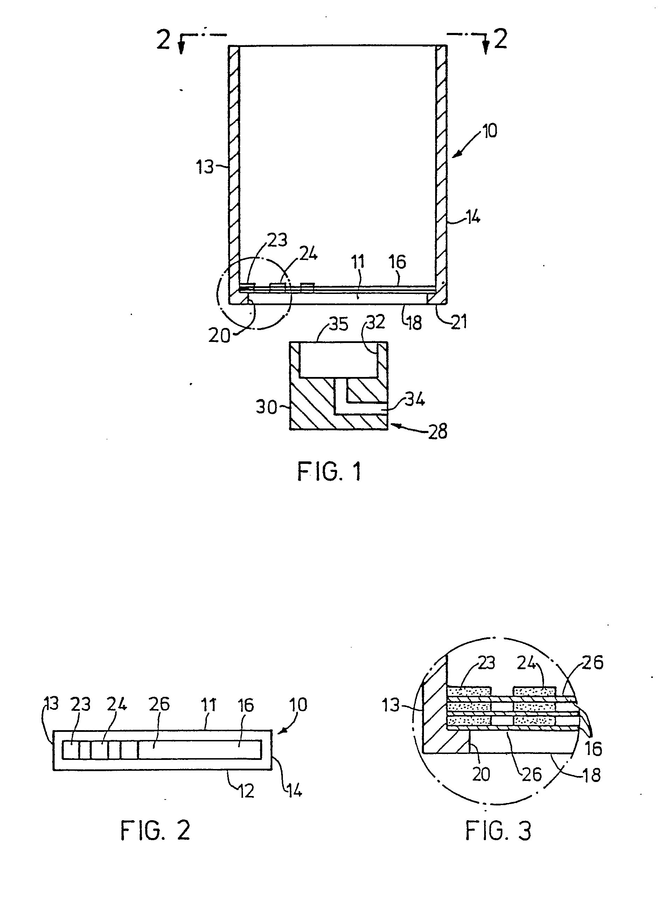

In Figs. 1 and 2 a cartridge or magazine 10 for test devices, such as reagent strips, is shown. Magazine 10, as illustrated, is open at the top and has rigid side walls 11 and 12 which together with rigid end walls 13 and 14 form an elongated rectangular shaped container having interior dimensions slightly larger than test devices 16 contained therein. The bottom of magazine 10 has an opening 18, the width of which is equal to the width of the interior dimensions of magazine 10 formed by the interior of side walls 11 and 12 and the length of which is slightly smaller than the interior dimensions of side walls 13 and 14. End member 20 and 21, which extend at right angles to side walls 13 and 14 of magazine 10, form detents or ledges at either end of opening 18 for retaining test devices 16 inside magazine 10. Thus, the bottommost test devices 16 inside magazine 10 is retained in magazine 10 solely by the detents or ledges formed by end members 20 and 21 and other test devices 16 are supported by the bottommost test device, resting one upon the other in parallel layered fashion.

The positioning of the test devices 16 inside magazine 10 is best illustrated in Fig. 3 which shows a lower corner of magazine 10 in enlarged cross section. This Figure illustrates how the test devices 16 rest one upon the other in parallel fashion inside magazine 10 with the bottommost test device 16 being held inside container 10 by the detents or ledges formed by the end members. The bottommost test device 16 is thus not supported at any other point except at its extreme ends by end members 20 and 21. Other test devices 16 inside magazine 10 rest one upon the other, as illustrated in Figs. 1 and 3, and are so positioned inside magazine 10 that reagent matrix areas 23 and 24, which are attached to a suitable substrate 26, face upward in a direction opposite opening 18.

Beneath magazine 10 and aligned with opening 18 is vacuum transport means or pickup head 28 having a housing 30 with an open chamber 32 at its top which is connected by means of conduit 34 to a vacuum source (not shown). Vacuum transport means 28 is capable of moving in at least two directions, up and down, and the dimensions of this member are such that upper surface 35 of vacuum transport means 28 can enter opening 18 of magazine 10 and contact the lower surface of substrate 26 of the bottommost test device 16 inside magazine 10.

In operation, magazine 10 normally is retained in a vertical position and loaded with multiple test devices 16 positioned inside magazine 10 in the manner illustrated by Figs. 1, 2 and 3. Test devices 16 are then removed one at a time from magazine 10, as required, by moving vacuum transport means 28 upward through opening 18 such that the top surface 35 of vacuum transport means 28 rests against substrate 26 of the bottommost test devices 16 inside magazine 10. By then applying a vacuum through conduit 34 the bottommost test device 16 can be held by the pressure exerted against chamber 32 of vacuum transport means 28 and said test device will move downward as vacuum transport means 28 moves downward. By pulling downwardly at the center of the bottommost test device 16 vacuum transport means 28 causes the flexible substrate 26 of said test device to flex and slightly distort into a U-shaped configuration in which the center of the bottommost test device 16 is lower than the ends of said test device held by the detents formed by end members 20 and 21. As vacuum transport means 28 continues to move downwardly the distortion of the bottommost test device 16 continues until the ends of said test device become released from the detents formed by end members 20 and 21 and test device 16 is thereby removed from magazine 10. Due to the fact that only the bottommost test device is distorted or flexed at any given time the other test devices remain inside magazine 10 as the bottommost test device 16 is withdrawn through opening 18. Once removed from magazine 10 a test device 16 can be transferred by vacuum transport means 28 or any other suitable means to a different location. Following such transfer the same or a different vacuum transport means 28 can be used to remove another test device 16 from magazine 10.

It should be understood that the materials used for construction of magazine 10 and vacuum transport means 28 are not critical and may be of any suitable rigid or semi-rigid material, such as metal, plastic, etc. Although the side walls of magazine 10 are normally continuous, retainers in the form of rod members may be used in place of continuous walls. As indicated above, the interior dimensions of magazine 10 must be such that multiple test devices 16 can be inserted into the magazine with sufficient tolerance to prevent binding between the test devices and the side walls. The detents or ledges retaining the bottommost test device 16 in magazine 10 need be only the minimum width necessary to provide support such that the weight of the test devices inside magazine 10 will not cause the bottommost or lowest test device 16 to pass through opening 18 until that test device is removed by vacuum transport means 28. Obviously, chamber 32 of vacuum transport means 28 may take many different configurations, such as a rectangular configuration, an eliptical configuration, a circular configuration, etc. In fact, chamber 32 need not be a single chamber, but may consist of multiple chambers. The top surface 35 of vacuum transport means 28 is preferably flat, but may be slightly curved so as to better adapt to the flexed configuration of the bottommost test device 16 as that test device is removed from magazine 10 through opening 18.

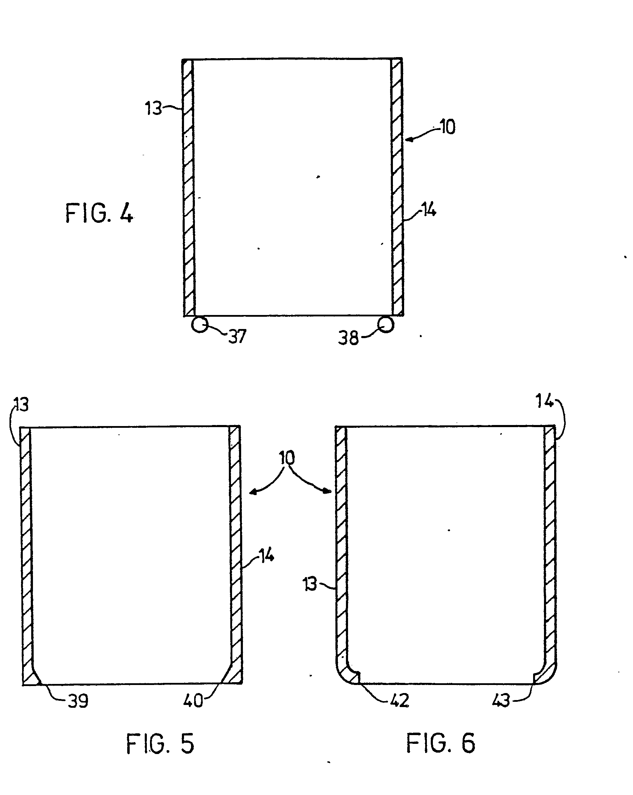

While the ledges or detents formed by end members 20 and 21 illustrated in Fig. 1 are preferred for retaining the bottommost test device 16 inside magazine 10, other detents for supporting test devices 16 may be used, including the alternatives illustrated in Figs. 4 through 6. In Fig. 4, for example, rod members 37 and 38 are employed to provide suitable support for the bottommost test device (not shown) placed in magazine 10. Rod members 37 and 38 are located at the bottom of end walls 13 and 14, respectively. In Fig. 5, tapered end members 39 and 40 are illustrated, whereas in Fig. 6 curved end members 42 and 43 are illustrated. These embodiments, together with other alternative embodiments, are within the scope of the present invention. The detents of the magazine 10 may also be pressure actuated (in a manner not shown), such that pressure applied to the detents will cause the detents to move slightly thereby facilitating the release of a single flexible member at a time from magazine 10.

While normally magazine 10 will be positioned in a vertical or upright position, such that the flexible members contained therein rest one upon the other by means of gravity, the concept of the present invention is applicable to pressure feed systems which by means of spring loading or other suitable pressure are capable of holding multiple flexible members in place against the detents or end members of the cartridge or magazine 10. Thus, if desired, test devices 16 could be held by suitable means, such as a spring member (not shown), against end members 20 and 21. For such loading magazine 10 would not need to be positioned in an upright position.

From the foregoing, it will be seen that this invention is well adapted to attain all of the ends and objects herein above set forth, together with other advantages which are obvious and which are inherent to the system. The present invention permits a single flexible object, such as a test device, to be removed from a container holding multiple flexible objects. While the invention has been described with particularity to test devices such as reagent strips, it will be understood that the system and handling apparatus may be used for the handling of other elongated flexible members such as thin metallic or plastic members. The pickup head and magazine of the present invention permit systems to be automated by assuring the retrieval and delivery of one flexible member at a time.

## Claims

1. Apparatus, comprising a magazine (10) retaining multiple flexible test strips (16) and means for removing a single flexible test strip (16) used in the detection of at least one constituent in a fluid from that magazine (10), the bottommost of the flexible test strips (16) being retained in the magazine (10) by ledge means (20, 21) positioned at two opposite ends of an elongated opening (18) located at one end of said magazine (10), said apparatus further comprising a pickup head (28) capable of passing through the opening (18) at said one end of the magazine (10) and making contact with the bottommost flexible test strip (16) inside the magazine (10), and means (34) for supplying a vacuum through said pickup head (28) such that the bottommost flexible test strip (16) is held against the pickup head (28), wherein the pickup head (28) causes the bottommost flexible test strip (16) to bend sufficiently as the pickup head (28) is removed from said opening (18) at the lower end of the magazine (10) to cause the withdrawal of said bottommost flexible test strip (16) from said magazine (10).

2. The apparatus of Claim 1, in which the ledge means (20, 21) comprise a pair of ledges (39, 40), each ledge extending at an angle greater than 90 degrees and less than 180 degrees from an endwall (13, 14) of said magazine, and the pickup head (28) is rectangular.

3. The apparatus of Claim 1 or 2, in which the flexible test strips (16) are elongated.

4. The apparatus of Claim 3, in which the elongated test strips (16) are reagent strips for detecting at least one constituent in a biological fluid.

5. The apparatus of any of Claims 1 to 4, in which the same pickup head (28) is used to sequentially remove adjacent flexible test strips (16) from the magazine (10).

6. The apparatus of any of Claims 1 to 4, in which different pickup heads (28) are used to sequentially remove adjacent flexible test strips (16) from the magazine (10).

7. The apparatus of any of Claims 1 to 6, in which the flexible test strips (16) are spring loaded.

8. A method of dispensing flexible test strips (16) using the apparatus according to Claim 1, comprising the steps of:

a) inserting multiple test strips (16) into the magazine (10) in such a way that said strips (16) rest one upon the other and the bottommost test strip (16) is retained in the magazine (10) at two opposite ends by said ledge means (20, 21);

b) contacting the bottommost test strip (16) with the pickup head (28) in such a way that the bottommost test strip (16) is held by vacuum pressure against the pickup head (28); and

c) moving the pickup head (28) away from the magazine (10), thereby causing the bottommost test strip (16) to flex at its end, and thereby remove said bottommost test strip (16) from the ledge means (20, 21) of said magazine (10).

9. The method of Claim 8, in which the same pickup head (28) sequentially removes bottommost test strips (16) from the magazine (10).

10. The method of Claim 8, in which different pickup heads (28) sequentially remove bottommost test strips (16) from the magazine (10).

## Revendications

1. Appareil, comprenant un magasin (10) retenant un grand nombre de bandelettes réactives flexibles (16) et des moyens destinés à retirer de ce magasin (10) une seule bandelette réactive flexible (16) utilisée dans la détection d'au moins un constituant d'un fluide, la plus basse des bandelettes réactives flexibles (16) étant retenue dans le magasin (10) par des moyens à rebords (20, 21) positionnés à deux extrémités opposées d'une ouverture allongée (18) située à une première extrémité dudit magasin (10), ledit appareil comprenant en outre une tête (28) de préhension capable de passer dans l'ouverture (18) de ladite première extrémité du magasin (10), et d'entrer en contact avec la plus basse bandelette réactive flexible (16) à l'intérieur du magasin (10) et des moyens (34) destinés à appliquer une dépression à travers ladite tête (28) de préhension de manière que la plus basse bandelette réactive flexible (16) soit maintenue contre la tête (28) de préhension, la tête (28) de préhension faisant suffisamment fléchir la plus basse bandelette réactive flexible (16) pour que, lorsque la tête (28) de préhension est retirée de ladite ouverture (18) de l'extrémité inférieure du magasin (10), elle provoque le retrait de ladite plus basse bandelette réactive flexible (16) dudit magasin (10).

2. Appareil selon la revendication 1, dans lequel les moyens à rebords (20, 21) comprennent deux rebords (39, 40), chaque rebord s'étendant sous un angle supérieur à 90° et inférieur à 180° à partir d'une paroi extrême (13, 14) dudit magasin, et la tête (28) de préhension est rectangulaire.

3. Appareil selon la revendication 1 ou 2, dans lequel les bandelettes réactives flexibles (16) sont allongées.

4. Appareil selon la revendication 3, dans lequel les bandelettes réactives allongées (16) sont des bandelettes à réactif destinées à la détection d'au moins un constituant d'un fluide biologique.

5. Appareil selon l'une quelconque des revendications 1 à 4, dans lequel la même tête (28) de préhension est utilisée pour retirer successivement du magasin (10) des bandelettes réactives flexibles adjacentes (16).

6. Appareil selon l'une quelconque des revendications 1 à 4, dans lequel différentes têtes (28) de préhension sont utilisées pour retirer successivement du magasin (10) des bandelettes réactives flexibles adjacentes (16).

7. Appareil selon l'une quelconque des revendications 1 à 6, dans lequel les bandelettes réactives flexibles (16) sont chargées par des ressorts.

8. Procédé de distribution de bandelettes réactives flexibles (16) à l'aide de l'appareil selon la revendication 1, comprenant les étapes qui consistent:

a) à introduire un grande nombre de bandelettes réactives (16) dans le magasin (10) de manière que lesdites bandelettes réactives (16) reposent les unes sur les autres et que la plus basse bandelette réactive (16) soit retenue dans le magasin (10), à deux extrémités opposées, par lesdits moyens à rebords (20, 21);

b) à faire entrer en contact la plus basse bandelette réactive (16) avec la tête (28) de préhension de manière que la plus basse bandelette réactive (16) soit maintenue par une dépression contre la tête (28) de préhension; et

c) à éloigner la tête (28) de préhension du magasin (10) de façon que la plus basse bandelette réactive (16) fléchisse, à son extrémité, et soit ainsi retirée des moyens à rebords (20, 21) dudit magasin (10).

9. Procédé selon la revendication 8, dans lequel la même tête (28) de préhension retire successivement du magasin (10) les plus basses bandelettes réactives (16).

10. Procédé selon la revendication 8, dans lequel différentes têtes (28) de préhension retirent successivement du magasin (10) les plus basses bandelettes réactives (16).

**Patentansprüche**

1. Vorrichtung, die ein Magazin (10) zur Aufnahme mehrerer flexibler Teststreifen (16) und eine Einrichtung zur Entfernung einzelner flexibler Teststreifen (16) umfasst, wobei die Teststreifen zum Nachweis mindestens einer Komponente in der Flüssigkeit aus dem Magazin (10) dienen, und der zuunterst gelegene flexible Teststreifen (16) in dem Magazin (10) mittels einer leistenartigen Einrichtung (20, 21), welche an zwei gegenüberliegenden Enden der länglichen Öffnung (18) an einem Ende des genannten Magazins (10) angebracht ist, zurückgehalten wird, wobei die Vorrichtung im weiteren einen Aufnehmerkopf (28) umfasst, welcher durch die Öffnung (18) an dem genannten einen Ende des Magazins (10) hindurchgeht und einen Kontakt mit dem zuunterst gelegenen flexiblen Teststreifen (16) innerhalb des Magazins (10) herstellt, sowie eine Einrichtung (34) zur Anwendung eines Vakuums durch den genannten Aufnehmerkopf (28), so dass der zuunterst gelegene flexible Teststreifen (16) gegen den Aufnehmerkopf (28) gehalten wird, wobei der Aufnehmerkopf (28) bewirkt, dass der zuunterst gelegene flexible Teststreifen (16) in ausreichendem Masse gebogen wird, während der Aufnehmerkopf (28) aus der genannten Öffnung (18) am unteren Ende des Magazines (10) entfernt wird, wobei der zuunterst gelegene flexible Teststreifen (16) aus dem Magazin (10) entfernt wird.

2. Vorrichtung nach Anspruch 1, dadurch gekennzeichnet, dass die leistenartige Vorrichtung (20, 21) eine Leistenpaar (39, 40) umfasst, wobei jede Leiste einen Winkel von über 90° und unter 180° mit der endständigen Wand (13, 14) des genannten Magazins bildet, und der Aufnehmerkopf (28) rechteckig ist.

3. Vorrichtung nach Anspruch 1 oder 2, dadurch gekennzeichnet, dass die flexiblen Teststreifen (16) länglich ausgebildet sind.

4. Vorrichtung nach Anspruch 3, dadurch gekennzeichnet, dass die länglichen Teststreifen (16) Reagenzstreifen zum Nachweis mindestens einer Komponente in einer biologischen Flüssigkeit darstellen.

5. Vorrichtung nach einem oder mehreren der Ansprüche 1 bis 4, dadurch gekennzeichnet, dass derselbe Aufnehmerkopf (28) dazu dient, nacheinander benachbarte flexible Teststreifen (16) aus dem Magazin (10) zu entfernen.

6. Vorrichtung nach einem oder mehreren der Ansprüche 1 bis 4, dadurch gekennzeichnet, dass verschiedene Aufnehmerköpfe (28) verwendet werden, um nacheinander benachbarte flexible Teststreifen (16) aus dem Magazin (10) zu entfernen.

7. Vorrichtung nach einem oder mehreren der Ansprüche 1 bis 6, dadurch gekennzeichnet, dass die flexiblen Teststreifen (16) unter Federdruck stehen.

8. Verfahren zum Verteilen flexibler Teststreifen (16) unter Verwendung der Vorrichtung nach Anspruch 1, gekennzeichnet durch:

(a) Einlegen mehrerer Teststreifen (16) in das Magazin (10) in einer Art und Weise, dass die genannten Teststreifen (16) aufeinander liegen und der zuunterst gelegene Teststreifen (16) in dem Magazin (10) an zwei einander gegenüberliegenden Enden mit Hilfe einer leistenartigen Einrichtung (20, 21) zurückgehalten wird;

(b) Kontaktieren des zuunterst gelegenen Teststreifens (16) mit dem Aufnehmerkopf (28) in einer Art und Weise, dass der zuunterst gelegene Teststreifen (16) mittels Vakuum gegen den Aufnehmerkopf (28) gehalten wird; und

(c) Entfernen des Aufnehmerkopfes (28) weg von dem Magazin (10), wodurch sich der zuunterst gelegene Teststreifen (16) an dessen Ende biegt und auf diese Weise der zuunterst gelegene Teststreifen (16) aus der leistenartigen Vorrichtung (20, 21) des genannten Magazins (10) entfernt wird.

9. Verfahren nach Anspruch 8, dadurch gekennzeichnet, dass derselbe Aufnehmerkopf (28) nacheinander die zuunterst gelegenen Teststreifen (16) aus dem Magazin entfernt.

10. Verfahren nach Anspruch 8, dadurch gekennzeichnet, dass verschiedene Aufnehmerköpfe (28) nacheinander die zuunterst gelegenen Teststreifen (16) aus dem Magazin (10) entfernen.

FIG. 1

FIG. 2

FIG. 3

FIG. 4

FIG. 5

FIG. 6